# FASCICULE DE BREVET EUROPEEN

(11) **EP 1 165 042 B1**
(45) Date de publication et mention de la délivrance du brevet: **02.02.2005**
(21) Numéro de dépôt: 00917139.8
(22) Date de dépôt: 06.04.2000
(51) Int. Cl.: A61K 9/00, A61K 31/192

(54) **SUSPENSION PHARMACEUTIQUE BUVABLE D'IBUPROFENE**
TRINKBARE PHARMAZEUTISCHE SUSPENSION VON IBUPROFEN
DRINKABLE IBUPROFEN PHARMACEUTICAL SUSPENSION

(30) Priorité: 06.04.1999 FR 9904251; 05.04.2000 FR 0004359
(43) Date de publication de la demande: 02.01.2002
(73) Titulaire: ETHYPHARM, 78550 Houdan (FR)
(72) Inventeur: LEBON, Christophe, F-28260 Rouvres (FR); GUERIN, Emmanuel, F-75013 Paris (FR); SUPLIE, Pascal, F-27400 Montaure (FR)
(74) Mandataire: Ahner, Francis
(86) Numéro de dépôt international: PCT/FR2000/000869
(87) Numéro de publication internationale: WO 2000/059467

(56) Documents cités:
- EP-A- 0 468 232
- EP-A- 0 559 463
- WO-A-95/17177

## Description

La présente invention concerne une composition pharmaceutique buvable d'Ibuprofène destinée à une administration orale facilitée.

L'administration orale de formes solides comme les comprimés peut s'avérer difficile voire dangereuse pour les enfants et les personnes âgées. qui préfèrent des comprimés à mâcher, des comprimés qui fondent dans la bouche ou dans une cuillère d'eau, des granules des poudres. des solutions ou des suspensions.

Les principes actifs incorporés dans ces types de formulations ont parfois un goût amer qui persiste longtemps et qui ne peut être masqué de façon efficace par l'addition d'un édulcorant ou d'un aromatisant. Or le goût et l'arrière-goût sont des critères importants pour l'acceptance du médicament par le malade.

Dans les suspensions buvables de principe actif amer déjà décrites dans l'art antérieur, le principe actif est généralement enrobé avec une substance lipidique, soit directement, soit après avoir été incorporé dans un noyau. Les substances lipidiques utilisées sont par exemple une huile végétale partiellement hydrogénée (EP 670 722). l'acide stéarique et/ou l'acide palmitique (FR 2 615 101). le tripalmitate de glycéryle (EP 664 701) ou un mélange de monostéarate de glycéryle et de cire d'abeille dans les proportions 9/1 (EP 769 948).

L'Ibuprofène est un principe actif au goût amer qui a fait l'objet de nombreuses études pour le formuler dans des compositions stables dans lesquelles son goût est masqué

Une solution de l'art antérieur consiste à enrober les granules contenant l'Ibuprofène avec un polymère destiné à masquer son goût amer Ce polymère est par exemple un phtalate (WO 91/16043), un vinyl-acétal (JP 91/83922), un phtalate acétate de cellulose (WO 95/05166) ou un copolymère d'acide méthacrylique (EP 524 180)

Une autre solution de l'art antérieur consiste à préparer une suspension aqueuse d'Ibuprofène. Cette suspension contient soit une substance lipidique et un tensio-actif (WO 94/25006. US 5 110 606 WO 96/22762, WO 94/05260), soit un agent de suspension, comme un polysaccharide (JP 98/182 449), un mélange de cellulose et de gomme xanthane (EP 556 057), un mélange de polyéthylèneglycol et de carboxyméthylcellulose de sodium (US 5 602 182), un mélange de gomme xanthane et d'amidon prégéiatinisé (EP 405 930), un méiange de gomme xanthane, de cellulose microcristalline et de carboxyméthylcellulose (EP 298 740).

WO 95/17177 décrit des compositions pharmaceutiques liquides visqueuses à base d'Ibuprofène qui consistent en une dispersion du principe actif dans une solution très visqueuse, dont le pH a été ajusté entre 1,0 et 5,0 et de préférence entre 3,0 et 4,0. Cette dispersion contient, en outre, un agent de sapidité, un agent mouillant, un agent édulcorant et un agent d'aromatisation.

L'objet de la présente invention est une suspension pharmaceutique buvable d'lbuprofène qui se présente sous une forme sèche, reconstituable sous forme liquide, ou qui est prête à l'emploi sous forme liquide, comprenant une phase liquide dans laquelle est dispersé l'Ibuprofène à l'état solide, caractérisée en ce que la fraction solubilisée de l'Ibuprofène dans la phase liquide est inférieure à 10 % en poids par rapport à la quantité totale d'Ibuprofène, de préférence 5% en poids, de préférence inférieure à 1% en poids par rapport au poids de la suspension.

Le principe actif est sous la forme de cristaux, de préférence des cristaux de dimension inférieure à 500 microns, ou formulés dans des granules. Les cristaux sont enrobés ou non d'une substance filmogène.

Le principe actif se trouve dispersé dans la phase liquide de façon à obtenir une suspension stable et homogène.

Les granules sont éventuellement enrobés d'un matériau destiné soit à assurer la libération modifiée du principe actif fixé sur un support neutre, soit à masquer le goût du principe actif, soit à éviter de solubiliser le principe actif dans la phase liquide de la suspension.

Les granules ont une taille finale comprise entre 50 et 1000 µm (microns) de préférence entre 200 et 600 µm (microns). Ils sont constitués d'un noyau inerte de taille sensiblement comprise entre 100 et 350 µm (microns) et de forme sphérique sur lequel est appliqué le principe actif au moyen d'un liant ou par simple adsorption si le principe actif est en solution.

Les granules sont par exemple obtenus par montage du ou des principes actifs sur un support neutre constitué de sucre et d'amidon.

Le montage du principe actif sur le support neutre peut être effectué selon plusieurs techniques, comme le poudrage, la pulvérisation d'une

Le montage du principe actif sur le support neutre peut être effectué selon plusieurs techniques, comme le poudrage, la pulvérisation d'une solution ou d'une suspension de principe actif, ou toute autre technologie classique connue de l'homme de l'art.

Le support neutre enrobé de principe actif est ensuite éventuellement enrobé d'un film polymérique constitué d'un seul polymère ou d'une combinaison de plusieurs polymères, ou enrobé d'une succession de différentes couches de polymère, en fonction de l'effet attendu. Cette étape peut être réalisée en utilisant les différents excipients et technologies bien connues de l'homme du métier. On utilisera de préférence des solvants aqueux.

La suspension pharmaceutique buvable selon l'invention est avantageusement constituée d'une phase liquide aqueuse ou constituée d'un mélange d'eau avec un co-soivant, par exemple une huile, le propylène glycol, la glycérine ou une solution de sorbitol.

La suspension selon l'invention peut également se présenter sous la forme d'un mélange sec d'excipients et de principe actif, pouvant être préparée extemporanément par simple addition d'eau. Le principe actif est alors dispersé par agitation manuelle, de façon à obtenir une formulation homogène et stable.

La suspension sèche est préparée en mélangeant le principe actif, sous la forme de cristaux ou de granules, et les adjuvants de suspension. Elle peut être granulée selon les différents techniques de granulation classiques bien connues de l'homme du métier, ou résulter du simple mélange physique des différents composants.

La poudre est ensuite répartie en flacons. Les flacons sont suffisamment grands pour que la quantité d'eau nécessaire à la reconstitution puisse être ajoutée.

La suspension pharmaceutique buvable selon l'invention contient au moins un agent de viscosité, au moins un agent de charge et au moins un agent conservateur.

L'agent de viscosité est choisi parmi les agents de viscosité pharmaceutiquement acceptables par exemple la gomme xanthane, l'hydroxypropylméthylcellulose, la méthylcellulose, la carageenane, la carboxyméthylcellulose, la cellulose microcristalline, la polyvinylpyrolidone et les carbomères.

L'agent de viscosité est avantageusement un carbomère. Les carbomères sont des polymères d'acide acrylique contenant des groupements interchaines allylsucrose et allylpenta-érythritol. On choisit de façon préférentielle un grade dont la synthèse ne nécessite pas l'utilisation de benzène comme solvant, comme le *carbomère 971P*. En outre, les carbomères présentent l'avantage d'être utilisés en faibles quantités.

L'agent de viscosité représente 0,1 à 10%, de préférence 0,1 à 2,5%, ou encore 0,1 à 1% en poids par rapport au poids de la suspension.

Dans toute la demande, dans l'expression "% en poids par rapport au poids de la suspension", on entend par suspension sous forme liquide éventuellement reconstituée.

L'agent de charge représente 10 à 70%, de préférence 30 à 55%, en poids par rapport au poids de la suspension. Il est choisi parmi le saccharose ou les agents non cariogènes comme le sorbitol, le xylitol, le mannitol, le lactitol ou les maltodextrines.

L'agent conservateur représente 0,05 à 3%, de préférence 0,1 à 1%, en poids par rapport au poids de la suspension.

Le conservateur ou le mélange de conservateurs permet de maintenir l'intégrité microbiologique de la suspension et de répondre aux exigences réglementaires, relatifs au dénombrement de germes totaux et spécifiques et à l'étude d'efficacité des conservateurs.

Un mélange d'agents conservateurs est choisi préférentiellement de façon à obtenir une synergie d'effets antibactériens.

Au moins un des agents conservateurs est choisi parmi les esters de parabens ou les sels correspondants.

La suspension buvable selon l'invention peut en outre comprendre un agent alcalinisant ou acidifiant de façon à ajuster le pH entre 2 et 6, de façon préférentielle entre 4 et 5,5.

La suspension buvable selon l'invention peut en outre contenir au moins un agent choisi parmi un agent alcalinisant ou acidifiant, un agent aromatisant, un agent anti-oxydant, un agent colorant, un agent lubrifiant et un édulcorant. aromatisant, un agent anti-oxydant, un agent colorant, un agent lubrifiant et un édulcorant.

L'édulcorant est choisi selon son pouvoir sucrant supérieur au saccharose tel que l'aspartame, la saccharine, le cyclamate sodique et leurs mélanges.

L'arôme peut être d'origine naturelle ou synthétique. Il représente 0,1 à 3% en poids par rapport au poids de la suspension.

Le colorant est un pigment naturel ou synthétique, choisi en fonction de l'arôme de suspension. Dans le cas d'un arôme fraise, on choisira préférentiellement le rouge de cochenille ou le rouge Ponceau 4R.

L'agent lubrifiant est par exemple le talc, l'oxyde d'aluminium ou la silice.

Les exemples suivants illustrent la présente invention.

### Exemple 1 : suspension de cristaux d'Ibuprofène.

Un lot correspondant à 300 litres de suspension soit 2000 flacons de 1 50 ml est préparé à partir de la formule suivante

| **COMPOSANTS** | **FORMULE DE FABRICATION** |
|---|---|
| Ibuprofène | 6.00 kg |
| Saccharose | 115.50 kg |
| Sorbitol à 70 % non cristallisable | 18.30 kg |
| Carbomères (Carbopol® 971 P) | 0.51 kg |
| Parabens (Nipasept® base) | 0.35 kg |
| Arôme fraise | 0.35 kg |
| Colorant Rouge Cochenille A | 0.010 kg |
| Hydroxide de sodium (solution 1 N) | 2.00 kg |
| Eau purifiée | 208.30 kg |

### Préparation n°1

Dans la cuve de 200 I, introduire l'eau (127 kg) à température ambiante, puis disperser par petites quantités le Carbopol® 971 P dans l'eau.
Homogénéiser avec une turbine défloculeuse pendant 1 heure à 1500 t/mn puis pendant 4 heures à 800 t/mn.

### Préparation n°2

Chauffer une cuve double enveloppe de 400 litres à 80°C et introduire l'eau (75 kg) à 80°C. Maintenir la température de l'eau entre 75°C et 80°C pendant la préparation.
Monter la turbine tripale Æ 200 mm sur l'agitateur RAYNERI DIRECT T 80.
Mettre en route et conserver l'agitation (200t/mn), tout au long de la préparation.
Disperser les conservateurs et l'arôme, attendre leur dissolution complète.

Ajouter le saccharose et attendre sa dissolution complète (vérifier la limpidité de la solution), ajouter le sorbitol liquide 70% puis rincer le contenant avec de l'eau (6,3 kg).
Ajouter le colorant et poursuivre l'agitation jusqu'à obtenir une solution limpide.
Refroidir la la double enveloppe et attendre que la température de la solution soit redescendue à 35°C.

Dans la cuve de 400 I, verser sous agitation (200t/mn), la préparation n°1 dans la préparation n°2. Homogénéiser (300 t/mn) pendant au moins 30 minutes.
Préparer une solution de NaOH 1N.
Sous agitation (300 à 500 t/mn), introduire progressivement une fraction de solution de NaOH 1N. Homogénéiser pendant 15 minutes.
Introduire sous agitation (500 t/mn) l'Ibuprofène et homogénéiser pendant 1 heure.
En maitenant l'agitation, ajouter le solde de la solution de NaOH 1N puis contrôler le pH (5,4). Ajuster si besoin le pH à 5,4 avec le solde de la solution de NaOH 1N.
Homogénéiser pendant 2 heures (300 t/mn).
Effectuer le réglage du volume unitaire (150 ml) et procéder au remplissage des flacons. Maintenir l'agitation dans la cuve de 400 litres et la cuve tampon pendant le remplissage des flacons.
Prélever des flacons en début, milieu et fin de remplissage.

### Résultats

| | début | milieu | fin |
|---|---|---|---|
| flacon 1 | 99 | 103 | 103,5 |
| flacon 2 | 98,5 | 101,2 | 101,2 |

La teneur ne varie pas de manière significative au cours du remplissage. La suspension reste homogène.

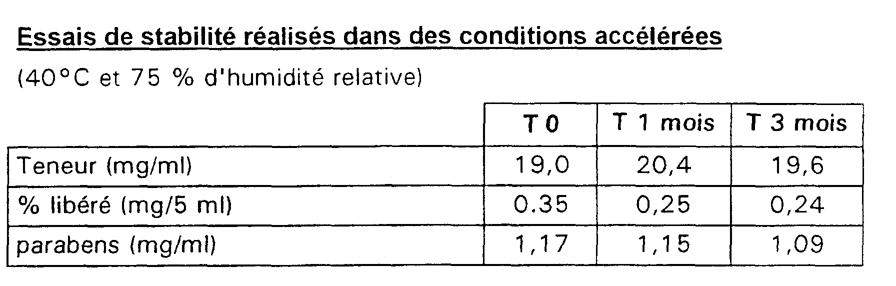

### Exemple 2 : suspension de cristaux d'Ibuprofène,

On réalise dans l'exemple ci-dessous un lot correspondant à 10 000 flacons de 200 ml :

| **COMPOSANTS** | |
|---|---|
| Ibuprofène cristaux | 40,0 kg |
| Saccharose | 770,666 kg |
| Sorbitol à 70 % non cristallisable | 122 kg |
| Carbopol®971 P | 3,466 kg |
| Nipagin® | 1,707 kg |
| Nipasol® | 0,253 kg |
| Hydroxybenzoate d'ethyle | 0,373 kg |
| Arôme fraise | 2,333 kg |
| Colorant Rouge Cochenille A | 0,067 kg |
| Hydroxyde de sodium | 0,533 kg |
| Eau purifiée | 1355 kg |

### Préparation

La suspension d'Ibuprofène selon le même mode opératoire, en adaptant le matériel à la taille du lot. Une cuve de 2000 litres équipée d'une double enveloppe est utilisée pour la préparation, ainsi qu'une cuve de 500 litres pour la préparation de la dispersion de Carbomère.

### Résultats

Des prélèvements (1 à 12) sont réalisés en cours de vidange de la cuve de 2000 litres.
2 lots de suspension sont réalisés et les teneurs sont les suivantes :

| | 1 | 2 | 3 | 4 | 5 | 6 | 7 | 9 | 10 | 12 |
|---|---|---|---|---|---|---|---|---|---|---|
| 58900 | 104 | | 107,0 | 107,3 | 106 | 103,3 | | 106 | 107,7 | |
| 58901 | 106,5 | 106,9 | 105,2 | 102,9 | | 108,3 | 104,8 | 109,7 | 101,3 | 97,9 |

Les teneurs sont homogènes, comprises dans les spécifications

### Exemple 3 : suspension de cristaux d'Ibuprofène

On procède comme dans l'exemple 1
La suspension est fabriquée selon la formule suivante :

| **Composant** | **Formule unitaire 150 ml*** | **% (p/p)** |
|---|---|---|
| Ibuprofène | 3.000 g | 1.71 % |
| Saccharose | 57.800 g | 32.93 % |
| Sorbitol à 70 % non cristallisable | 9.150 g | 5.21 % |
| Carbomères (Carbopol®971 P) | 0.260 g | 0.15 % |
| Parabens (Nipasept® base) | 0.175 g | 0.10 % |
| Arôme fraise | 0.175 g | 0.10 % |
| Colorant Rouge Cochenille A | 0.005 g | 0.003 % |
| Hydroxyde de sodium | 0.040 | 0.02 % |
| Eau purifiée | 104.895 g | 59.78 % |

Les parabens totaux représentant 0,1 % (p/p) de la suspension sont un mélange de trois esters de parahydroxybenzoates dans les proportions suivantes :
. 73 % Methylparaben
. 16 % Ethylparaben
. 11 % Propylparaben

Cette suspension est soumise à des conditions de stockage accélérées (40°C et 75 % d'humidité relative),
Les résultats obtenus sont résumés dans le tableau suivant.

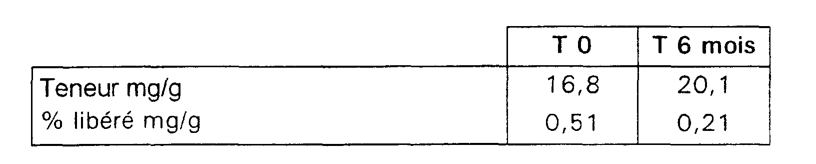

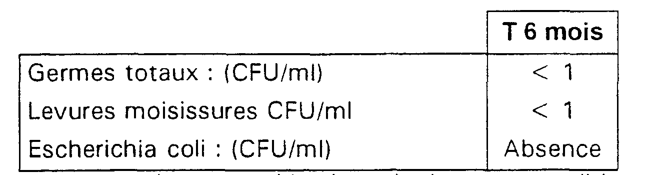

La suspension est stable six mois dans ces conditions.

### Essais d'efficacité des conservateurs

Un essai microbiologique est réalisé selon la méthode décrite dans la Pharmacopée Européenne 3ème éd.

L'essai de la suspension de cristaux est réalisé contre une suspension placebo de même composition mais ne contenant aucun conservateur.
- suspension avec conservateurs 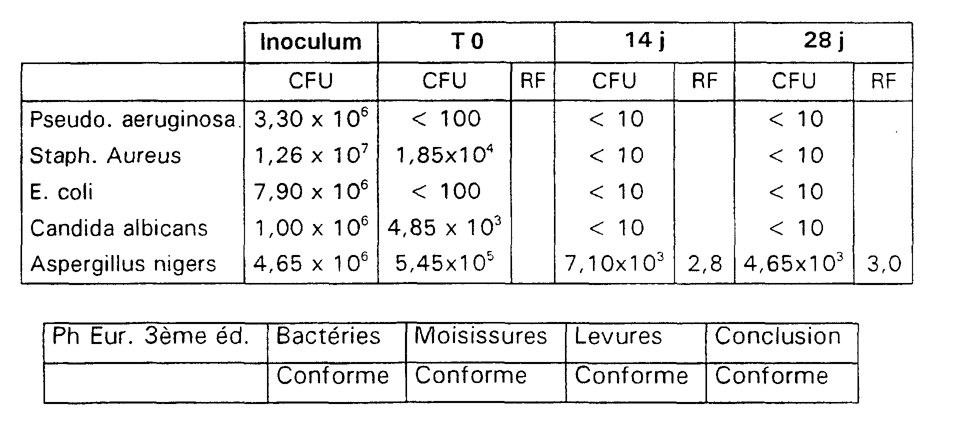
- suspension sans conservateur

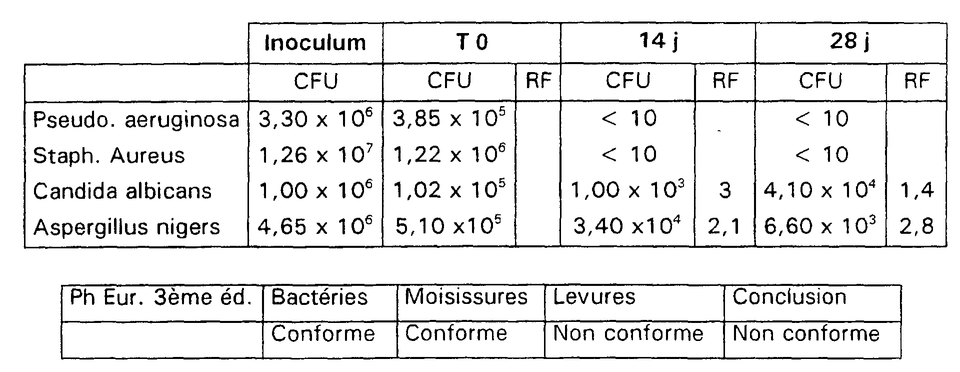

L'essai répond aux critères de la Pharmacopée Européenne 3ème éd. La concentration de 0,1% (p/) en parabens totaux permet une protection efficace de la suspension.

### Exemple 4 : suspension de granules d'Ibuprofène contenant 20 mg/ml d'Ibuprofène

### Formule des microgranules :

| **Excipients** | **Quantités (g)** | **% (p/p)** |
|---|---|---|
| Ibuprofène | 1,19 | 9,2 |
| Neutres | 4,64 | 35,7 |
| Eudragit E 100 ® | 0,071 | 0,55 |
| Eudragit NE30D ® | 0,060 | 0,46 |
| Silice colloidale | 0,006 | 0,04 |
| Talc | 0,024 | 0,18 |
| Alcool 95° (solvant) | 0,52 | - |

Les microgranules sont fabriqués par pulvérisation de couches successives d'actifs puis de dispersion d'enrobage permettant de masquer l'amertume du principe actif.

| Formule du sirop : | |
|---|---|
| **composants** | **% (p/p)** |
| Carbopol® 971 P | 0,14 |
| Eau purifiée | q.s. 100 % |
| Granules d'Ibuprofène | 1,7 |
| NaOH(1N) | q.s. pH = 5,5 |
| Sorbitol à 70 % | 3,4 |
| Saccharose cristaux | 30,8 |
| Nipasept® | 0,1 |
| Arôme fraise | 0,1 3 |

On ajoute les granules d'Ibuprofène titrés à 200 mg/g pour obtenir une suspension à 20 mg/ml d'Ibuprofène.
Le sirop est fabriqués selon les exemples précédents.

### Exemple 5 : suspension sèche de microgranules

Les microgranules sont préparés comme dans l'exemple 4

### 2) Formule du mélange adjuvant sec :

| **Excipients** | **Quantités (g)** | **% (p/p)** |
|---|---|---|
| Carbopol® 971 P | 46,0 | 2,62 |
| Saccharose | 1529 | 87,14 |
| Sorbitol | 153,0 | 8,72 |
| Citrate trisodique | 10,0 | 0,57 |
| Rouge de cocchenille | 0,6 | 0,03 |
| Arôme fraise | 11,0 | 0,63 |
| parabens totaux | 5,0 | 0,29 |
| Masse sèche totale | 1745,6 | 100% |
| H₂O (liquide de mouillage) | 120,0 | |

Les différents composants secs sont mélangés pendant 15 minutes dans un mélangeur cubique.

Les excipients sont ensuites granulés par ajout d'eau purifiée dans le mélange, pendant un temps suffisant. Les granulés sont séchés à 35°C pendant 3heures, puis calibrés sur une grille de maille 1,25 mm.

### Préparation finale : suspension d'lbuprofène :

| | |
|---|---|
| microgranules (g) | 6 |
| mélange adjuvant sec (g) | 7 |
| Quantité d'eau à ajouter (ml) | QSP 60 ml |
| Ibuprofène par flacon de 60 ml | 1,2 g |

On ajoute une quantité de sparklets et de granulés selon la proportion définie dans le tableau ci-desus.

La suspension est préparée par addition d'eau dans le flacon.
Par agitation énergique, les granules sont dispersés dans la phase liquide. Après 1 à 2 minutes au repos, la suspension est réagitée. Les granules sont alors répartis de façon homogènes dans la phase liquide et semaintiennent en suspension.

### Exemple 6 : suspension de microgranules d'Ibuprofène

| **COMPOSANTS** | **FORMULE UNITAIRE** **flacons 150 ml** | |
|---|---|---|
| | **gramme** | % |
| Microgranules d'Ibuprofène | 15 | 8,5 |
| Carbopol® 971 P | 0,2 | 0,11 |
| Saccharose | 59,2 | 33,75 |
| Sorbitol | 6,6 | 3,75 |
| Parabens totaux | 0,06 | 0,1 |
| Arôme fraise | 0,2 | 0,1 |
| Colorant | < 0,01 | < 0,01 |
| Na OH IM | qs ph 5,5 | qs ph 5,5 |
| Eau purifiée | qs | qs |

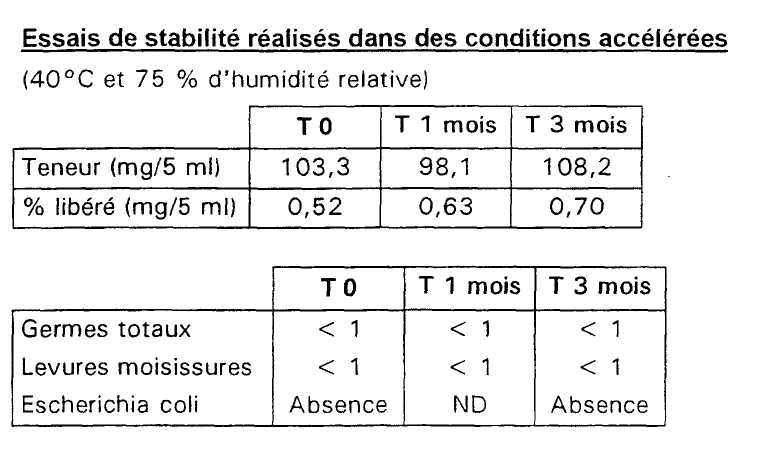

## Revendications

1. Suspension pharmaceutique buvable d'Ibuprofène qui se présente sous une forme sèche, reconstituable sous forme liquide, ou qui est prête à l'emploi sous forme liquide, comprenant une phase liquide dans laquelle est dispersé l'Ibuprofène à l'état solide, constituée
- d'un agent de viscosité du type carbomère,
- d'un agent de charge tel que le saccharose ou un polyol,
- d'un agent alcalinisant ou acidifiant,
- d'un agent aromatisant,
- d'un agent conservateur,
- d'un agent colorant, et
- d'Ibuprofène sous la forme de cristaux ou formulé dans des granules dispersé dans la phase liquide,
**caractérisée en ce que**
- la proportion de carbomère est comprise entre 0,1 et 2,5 % en poids par rapport au poids de la suspension,
- la proportion de l'agent alcalinisant ou acidifiant est choisie pour ajuster le pH entre 4 et 5,5, et
- la proportion de l'agent de charge est comprise entre 30 et 55 % en poids par rapport au poids de la suspension.

2. Suspension pharmaceutique buvable selon la revendication 1, **caractérisée en ce que** la proportion en carbomère est comprise entre 0,1 et 1 % en poids par rapport au poids de la suspension.

3. Suspension pharmaceutique buvable selon la revendication 1, **caractérisée en ce que** la granulométrie des cristaux est inférieure à 500 µm.

4. Suspension pharmaceutique buvable selon l'une des revendications 1 ou 2 **caractérisée en ce que** les granules sont enrobés d'un matériau polymérique destiné à masquer le goût de l'Ibuprofène ou à assurer la libération modifiée de l'Ibuprofène.

5. Suspension pharmaceutique buvable selon la revendication 3 **caractérisée en ce que** la granulométrie des granules est comprise entre 50 et 1000 µm.

6. Suspension pharmaceutique buvable selon l'une des revendications précédentes, **caractérisée en ce que** la phase liquide est aqueuse ou constituée d'un mélange d'eau et d'un co-solvant miscible.

7. Suspension pharmaceutique buvable selon l'une des revendications précédentes, **caractérisée en ce qu'**elle contient du saccharose ou un agent non cariogène, choisi parmi le sorbitol, le xylitol, le mannitol ou les maltodextrines.

8. Suspension pharmaceutique buvable selon l'une des revendications précédentes, **caractérisée en ce qu'**elle contient un mélange de conservateurs antibactériens dont l'un au moins est un ester de parabens, ledit mélange représentant 0,05 à 3%, de préférence 0,1 à 1%, en poids par rapport au poids de la suspension.

9. Suspension pharmaceutique buvable selon l'une des revendications précédentes, **caractérisée en ce que** l'agent aromatisant représente 0,1 à 3% en poids par rapport au poids de la suspension.

## Patentansprüche

1. Trinkbare pharmazeutische Suspension von Ibuprofen, die in trockener Form vorliegt, welche als flüssige Form wiederherstellbar ist, oder die gebrauchsfertig in flüssiger Form vorliegt, umfassend eine flüssige Phase, in der Ibuprofen in festem Zustand dispergiert ist, bestehend aus
- einem Viskositätsmittel vom Carbomer-Typ,
- einem Füllstoff, wie Saccharose oder einem Polyol,
- einem alkalinisierenden oder ansäuernden Mittel,
- einem Geschmacksmittel,
- einem Konservierungsmittel,
- einem Färbemittel und
- Ibuprofen in kristalliner Form oder in Granalien formuliert, welches in der flüssigen Phase dispergiert ist, .
**dadurch gekennzeichnet, dass**
- der Carbomer-Anteil zwischen 0,1 und 2,5 Gewichts-% einschließlich liegt, bezogen auf das Gewicht der Suspension,
- der Anteil des alkalinisierenden oder ansäuernden Mittels so ausgewählt ist, dass der pH zwischen 4 und 5,5 eingestellt ist, und
- der Anteil des Füllstoffs zwischen 30 und 55 Gewichts-% einschließlich liegt, bezogen auf das Gewicht der Suspension.

2. Trinkbare pharmazeutische Suspension nach Anspruch 1, **dadurch gekennzeichnet, dass** der Carbomer-Anteil zwischen 0,1 und 1 Gewichts-% einschließlich liegt, bezogen auf das Gewicht der Suspension.

3. Trinkbare pharmazeutische Suspension nach Anspruch 1, **dadurch gekennzeichnet, dass** die Korngröße der Kristalle unter 500 µm liegt.

4. Trinkbare pharmazeutische Suspension nach einem der Ansprüche 1 oder 2, **dadurch gekennzeichnet, dass** die Granalien mit einem Polymermaterial umhüllt sind, das dazu bestimmt ist, den Geschmack von Ibuprofen zu maskieren oder die modifizierte Freisetzung des Ibuprofens sicherzustellen.

5. Trinkbare pharmazeutische Suspension nach Anspruch 3, **dadurch gekennzeichnet, dass** die Korngröße der Granalien zwischen 50 und 1000 µm einschließlich liegt.

6. Trinkbare pharmazeutische Suspension nach einem der vorangehenden Ansprüche, **dadurch gekennzeichnet, dass** die flüssige Phase wässrig ist oder aus einer Mischung von Wasser und einem mischbaren Hilfslösungsmittel zusammengesetzt ist.

7. Trinkbare pharmazeutische Suspension nach einem der vorangehenden Ansprüche, **dadurch gekennzeichnet, dass** sie Saccharose oder ein nicht die Kariesbildung förderndes Mittel enthält, das aus Sorbit, Xylit, Mannit oder den Maltodextrinen ausgewählt ist.

8. Trinkbare pharmazeutische Suspension nach einem der vorangehenden Ansprüche, **dadurch gekennzeichnet, dass** sie eine Mischung von antibakteriellen Konservierungsmitteln enthält, von denen mindestens eines ein Ester von Parabenen ist, wobei die Mischung 0,05 bis 3, vorzugsweise 0,1 bis 1 Gewichts-% darstellt, bezogen auf das Gewicht der Suspension.

9. Trinkbare pharmazeutische Suspension nach einem der vorangehenden Ansprüche, **dadurch gekennzeichnet, dass** das Geschmacksmittel 0,1 bis 3 Gewichts-% darstellt, bezogen auf das Gewicht der Suspension.

## Claims

1. Oral pharmaceutical ibuprofen suspension which is provided in a dry form, reconstitutable in liquid form, or which is ready for use in liquid form comprising a liquid phase in which ibuprofen in a solid state is dispersed, consisting
- of a carbomer-type viscosity agent,
- of a filler such as sucrose or a polyol,
- of an alkalinizing or acidifying agent,
- of a flavouring agent,
- of a preservative,
- of a colouring agent, and
- of ibuprofen in the form of crystals or formulated in granules dispersed in the liquid phase,
**characterized in that**
- the proportion of carbomer is between 0.1 and 2.5% by weight relative to the weight of the suspension,
- the proportion of alkalinizing or acidifying agent is chosen so as to adjust the pH to between 4 and 5.5, and
- the proportion of filler is between 30 and 55% by weight relative to the weight of the suspension.

2. Oral pharmaceutical suspension according to Claim 1, **characterized in that** the proportion of carbomer is between 0.1 and 1% by weight relative to the weight of the suspension.

3. Oral pharmaceutical suspension according to Claim 1, **characterized in that** the size of the crystals is less than 500 µm.

4. Oral pharmaceutical suspension according to either of Claims 1 and 2, **characterized in that** the granules are coated with a polymer material intended to mask the taste of the ibuprofen or to allow the modified release of the ibuprofen.

5. Oral pharmaceutical suspension according to Claim 3, **characterized in that** the size of the granules is between 50 and 1 000 µm.

6. Oral pharmaceutical suspension according to one of the preceding claims, **characterized in that** the liquid phase is aqueous or consists of a mixture of water and of a miscible cosolvent.

7. Oral pharmaceutical suspension according to one of the preceding claims, **characterized in that** it contains sucrose or a noncariogenic agent chosen from sorbitol, xylitol, mannitol or maltodextrins.

8. Oral pharmaceutical suspension according to one of the preceding claims, **characterized in that** it contains a mixture of antibacterial preservatives at least one of which is an ester of parabens, said mixture representing 0.05 to 3%, preferably 0.1 to 1%, by weight relative to the weight of the suspension.

9. Oral pharmaceutical suspension according to one of the preceding claims, **characterized in that** the flavouring agent represents 0.1 to 3% by weight relative to the weight of the suspension.
